(19) Europäisches Patentamt

European Patent Office

Office européen des brevets

(11) Publication number: **0 222 709 B1**

## EUROPEAN PATENT SPECIFICATION

(45) Date of publication of patent specification: **22.05.91**  (51) Int. Cl.⁵: **A61M 1/34, A61M 1/16**

(21) Application number: **86830312.4**

(22) Date of filing: **29.10.86**

(54) Blood-purifying apparatus.

(30) Priority: **12.11.85 IT 6795585**

(43) Date of publication of application:
**20.05.87 Bulletin 87/21**

(45) Publication of the grant of the patent:
**22.05.91 Bulletin 91/21**

(84) Designated Contracting States:
**AT BE CH DE ES FR GB GR IT LI LU NL SE**

(56) References cited:
**EP-A- 0 044 694**
**EP-A- 0 165 519**
**DE-A- 2 901 628**
**US-A- 4 372 846**

(73) Proprietor: **SORIN BIOMEDICA S.p.A.**
**Strada per Crescentino**
**I-13040 Saluggia (Vercelli)(IT)**

(72) Inventor: **Gervasio, Renzo**
**Via B. Cafasso 21**
**I-10132 Torino(IT)**

(74) Representative: **Bosotti, Luciano et al**
**c/o Jacobacci-Casetta & Perani S.p.A. Via**
**Alfieri, 17**
**I-10121 Torino(IT)**

Rank Xerox (UK) Business Services

**Description**

The present invention relates to blood-purifying apparatus used for treating patient affected by renal failure, and relates particularly to the control (monitoring) of the ultrafiltration, both in a system of the type with haemofiltration alone and in a system of the type with combined haemodialysis-haemofiltration.

It is known that blood purification is carried out by bringing the blood itself into contact with a porous membrane and, by various processes, causing the toxic substances to leave it through the membrane. There are essentially two such processes: haemodialysis and haemofiltration. In both processes, the porous membranes are preferably walls of hollow fibres joined into a bundle in a container, and the blood flows within these fibres.

In haemodialysis, the blood is introduced into the fibres at a certain pressure, while an aqueous washing solution is passed around the outside of the fibres in counterflow, at a lower pressure; the blood purification occurs essentially by diffusion in that the impurities pass through the pores of the fibres into the washing solution which carries them away.

The walls of the fibres have very small pores whereby only impurities of low molecular weight can be removed. Urea generally passes well, vitamin B12 poorly and impurities of higher molecular weight not at all. Impurities of average-high molecular weight thus remain in the blood with this process.

In haemofiltration, the passage of the impurities through the porous membrane occurs solely by convection since the impurities are transported by the plasma; in fact, a pneumatic low pressure is created outside the fibres into which the blood is introduced. Hence, the name of ultrafiltration given to the action of this filter. In this case, the pores of the fibre walls are considerably larger than in the haemodialysis process, whereby impurities of average-high molecular weight are removed. Naturally, this process depletes the blood of a good part of the plasma whereby it is necessary to introduce a replacement liquid which compensates for this loss. Generally, during the treatment, up to about 40 litres of liquid are removed from the blood, and up to about 37 litres of replacement liquid are introduced. In such a case, therefore, it is necessary strictly to control both the quantity of liquid removed (ultrafiltrate) and the quantity of replacement liquid.

From what has been explained above, it can be inferred that haemodialysis purifies the blood of impurities of low molecular weight, while impurities of high molecular weight are removed by haemofiltration.

The combination of the two types of purification has been proposed and two solutions have arisen: haemodiafiltration, which uses a single filter of intermediate porosity to fulfil the two functions of dialysis and filtration together, and combined haemodilysis-haemofiltration, a method recently proposed by Ghezzi et al. (III Conference on Neurology and Dialysis, Bardonecchia, 24-25/3/83), which essentially combines the two functions of haemodialysis and haemofiltration but keeps them separate, however.

It provides a haemofilter located in series and upstream of a normal haemodialyser; all the convective component is entrusted to the first and the diffusion component of the purification to the second. Between the two is interposed a post-dilution, that is, an introduction of replacement liquid into the filtration residue. Even the monitoring of the two functions is carried out separately.

The problem of monitoring the ultrafiltration, both in the method with haemofiltration alone and in that with combined haemodialysis-haemofiltration, is of basic importance. One is in fact dealing with the precise detection of the quantity of liquid removed from the blood and the quantity of replacement liquid introduced in such manner that their difference is exactly the loss in body weight established for the patient.

The ultrafiltration monitoring devices known in the art act essentially through pressure regulation dependent on balances to remove or introduce the desired quantities of liquid. However, such devices are very delicate and complicated, require considerable operating experience and often display poor reliability.

Monitoring arrangements of that kind, or strictly related thereto are disclosed, for instance, in DE-A-2 901 628, EP-A-0 044 694, US-A-4 372 846 or EP-A-0 165 519, this latter document forming part of the state of the art only under Art. 54 (3) EPC. More specifically, the present invention relates to apparatus for the purification of blood having those features called for in the preamble of Claim 1, which is known, e.g. from EP-0 044 694.

The object of the present invention is an ultrafiltration control device which avoids the disadvantages outlined above and other disadvantages and which is conceived in accordance with criteria of maximum simplicity and reliability, while not leaving any margins for accidental errors which would compromise the process.

The main subject of the present invention is apparatus having the further features set forth in Claim 1.

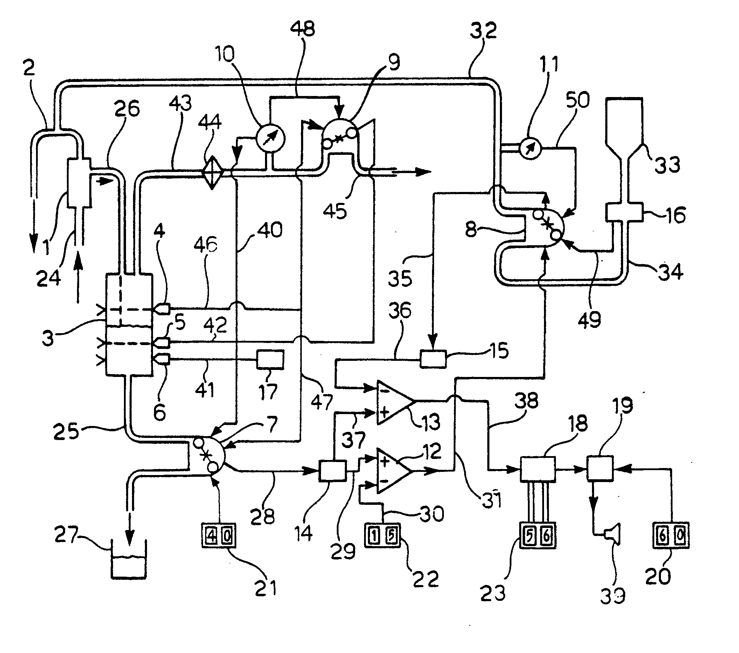

These and other characteristics of the present invention will become clearer from the following description of one preferred embodiment thereof, given by way of non-limiting examples, taken in connection with the appended drawing which is a partial block-schematic drawing of the hydraulic and electrical circuits making up the control device.

In the drawing, a filter for haemofiltration is generally indicated 1 and may have the characteristics of a filter for haemofiltration alone or those of a filter for combined haemodialysis-haemofiltration. A duct 2 connects it to the patient in the first case and to a filter for haemodialysis in the second case.

A reservoir, indicated 3, for collecting the ultrafiltrate, that is, the aqueous part of the blood containing the impurities, is provided with sensors, which may be ordinary photoelectric cells, arranged to indicate:

- the sensor 4 the maximum level of the liquid in the reservoir 3;
- the sensor 5 the minimum level of the liquid in the reservoir 3;
- the sensor 6 the presence of blood in the ultrafiltrate in the reservoir 3. This sensor is activated when the ultrafiltrate has a colour which tends even slightly to red or (or simultaneously) is slightly turbid.

Two volumetric suction-pressure pumps are indicated 7 and 8, and an air pump is indicated 9. The function of these pumps will be explained below during the description of the operation of the device.

Two manometers, indicated 10 and 11, measure the pressure of the fluid in the duct connected to each of these, and are arranged to output a signal indicative of anomalous behaviour of the pressure in order to control the stopping or the starting of the pumps connected thereto.

Two ordinary algebraic adders are indicated 12 and 13 and are arranged to add the signals fed to them algebraicly and to output a signal indicative of the result of the operation carried out by them.

Two flow gauges, which may be, for example, two ordinary tachometers, are indicated 14 and 15. A detector for air in the liquid which passes through it is indicated 16, and an optical/acoustic indicator acting as a malfunction alarm is indicated 17.

An ordinary integrator, indicated 18, is arranged to output a digital signal and a comparator, indicated 19, is arranged to compare the signals fed to it by the integrator 18 and by a presetting regulator 20 of any known type. Two further presetting regulators are indicated 21, 22 and are of the same type as the previous one. An ordinary dial arranged to receive a digital signal is indicated 23.

The device operates as follows.

The blood to be purified enters the haemofiltration filter 1 through a duct 24. At the same time, the suction part of the pump 7 creates a low pressure in the filter 1 through a duct 25, the reservoir 3 and a duct 26, a low pressure which causes the ultrafiltrate to flow through the duct 26 and fall into the reservoir 3 from which it will flow, still under the suction action of the pump 7, through the duct 25 and the pump itself, which with its pressure part will convey it to a collecting reservoir 27. The reservoir 27 may have any type of instrument for measuring the liquid expelled.

The speed of the pump is determined externally by means of the regulator 21 in which the quantity of liquid to be extracted per unit of time is set, this quantity being proportional to the speed of the pump. An indication of the speed of the pump is transmitted, for control, through a duct 28 to the ultrafiltrate flow gauge 14.

The signal from the gauge 14 is transmitted through a lead 29 to the algebraic adder 12 which is also fed through a lead 30 with the signal from the regulator 22 which always presents the fixed weight loss for the patient in kg/h, in value which is fixed externally at the start of the operation. The adder 12 adds the signals present on the two leads 29 and 30 algebraicly (the signal on 30 is obviously negative); the result of this sum is conveyed by a lead 31 to the pump 8 which, through a duct 32, conveys the replacement liquid which will be added at 2 to the blood purified in the filter 1. The replacement liquid is returned by the pump 8 from a reservoir 33 through a duct 34. The quantity of liquid conveyed is proportional to the speed of the pump 8, a speed which is regulated by the signal in the lead 31. The pump 8 sends a signal to the flow gauge 15 for the replacement liquid through a lead 35; in its turn, the gauge 15 outputs a signal to the algebraic adder 13 on a lead 36, which will be subtracted from the signal coming from the ultrafiltrate flow gauge 14 on a lead 37; the algebraic sum of the two signals will indicate the weight loss of the patient at the moment in question. The signal indicative of this sum is transmitted through a lead 38 to the integrator 18 which will provide on the dial 23 a numerical signal indicating the progressive loss in body weight.

A signal from the integrator 18 goes to the comparator 19 which also receives the signal of the total weight which must be lost during the entire operation, this signal coming from the preset regulator 20. When the two signals from the integrator 18 and the regulator 20 coincide, the comparator outputs a signal which is converted into an acoustic signal by an ordinary loudspeaker 39.

This is basically how the operation progresses. During its course, however, numerous checks and adjustments take place in real time, in order to ascertain any anomalous behaviour or infiltration of air into the circuits and to stop the machine in cases of malfunction.

If the ultrafiltrate reaches the minimum level in the reservoir 3, this indicates that an undesirable quantity of air is present in the reservoir itself.

The inconvenience usually occurs when the filter 1 cannot discharge the quantity of ultrafiltrate required by the pump 7; it is then necessary to stop it, expel the air, and allow time for the ultrafiltrate to reach a normal operating level. In order to start this series of operations, the minimum level sensor 5 sends a signal to the pump 7 through a lead 42 and a collector lead (bus) 40 to stop it; simultaneously, the same signal 42 is sent to the air pump 9 to start it. This draws the air from the reservoir 3 through a duct 43. The manometer 10 is connected to the duct 43 and indicates the suction pressure, and a filter 44 is also connected to ensure only the passage of air in the duct itself. The air extracted is then exhausted by the pump 9 through a duct 45. As a result of the suction of the pump 9, the ultrafiltrate accumulates in the reservoir 3 until it reaches a level that will be indicated by the maximum level sensor 4 which in its turn sends a signal through a lead 46. This signal stops the air pump 9 and, through a lead 47, simultaneously starts the pump 7, thus restoring normal operation of the apparatus.

The manometer 10 which measures the suction pressure in the duct 43 has a safety device which, whenever the suction pressure falls below a predetermined safety level, outputs a signal on 48 to stop the pump 9 and a signal through the bus 40 to stop the pump 7. This resembles in general an external control for the operation.

If there are traces of blood in the ultrafiltrate collected in the reservoir 3 during the operation, indicating that at least one fibre of the filter 1 has a leak, the indicator 6 indicates this presence and emits a signal which passes through a lead 41 and the bus 40 to the pump 7 on which it acts as a stop signal; this same signal also operates the alarm device 17 through the lead 41 and calls for external control by the operator.

The air detector 16 connected to the duct 34 which conveys replacement liquid from the reservoir 33 to the pump 8 comes into operation when air is present in the liquid itself. It them emits a signal through a lead 49, stopping the pump 8 which emits an alarm signal to call for external intervention.

The pressure of the replacement liquid conveyed by the pump 8 in the duct 32 is measured constantly by the manometer 11. It may happen that this pressure becomes too high, for example, when the solution in the duct 32 can no longer flow towards the duct 2 and the filter 1 because of a blockage in the filter itself. When the pressure surpasses a certain predetermined level, a maximum device with which the manometer is provided comes into operation; it then outputs a signal through a lead 50, stopping the pump 8 and giving an external alarm.

The basis of the invention is obvious from what has been explained above: the control of the flow of liquids in play - ultrafiltrate and replacement liquid - is entrusted to the controlled action of the volumetric pumps, that is, pumps in which the ratio between the speed of the pump and the flow obtained is constant. This proposition is rendered operative by rigorous controls established in order to avoid absolutely the undesirable presence of air in the fluid circuits.

## Claims

1. Apparatus for the purification of blood using the process of haemofiltration or of combined haemodialysis-haemofiltration, wherein the required balance between the liquid (3) removed from the blood (ultrafiltrate) and the replacement liquid (33) is achieved by regulation of the flow of the liquids with the use of first (7) and second (8) volumetric pumps for the ultrafiltrate and the replacement liquid, respectively, whose speeds of operation are regulated, characterised in that means (5,40,42,9) are provided for positively controlling, in real time, the amount of air present in the liquid circuit, wherein first means are provided for controlling, in real time, the amount of air present in the ultrafiltrate and are arranged to separate the air from the liquid body of the ultrafiltrate itself, second means (5) are provided for detecting the reduction in the liquid body due to the increase of the gaseous part, for deactivating the first volumetric pump (7) for the ultrafiltrate, and for activating a suction pump (9) for removing the excess gases, and finally third means (4) are provided for detecting when the liquid body reaches the normal quantity and for stopping the suction pump (9) as a consequence, at the same time restarting the first volumetric pump (7) for the ultrafiltrate.

2. Apparatus according to Claim 1, characterised in that the speed of the second volumetric pump (8) arranged to convey the replacement liquid is regulated in real time by means (12) for continuously computing the quantity of replacement liquid necessary at any instant by integration of the ultrafiltrate and for outputting consequent signals which effect the regulation of the rate of the second volumetric pump (8).

3. Apparatus according to any one of the preceding claims, characterised in that means (10) for controlling the presence of air in the ultrafiltrate circuit are provided which are arranged to detect the lowering of the air pressure and are also arranged to stop the air suction pump (9) and the first volumetric pump (7) for the ultrafiltrate consequently.

4. Apparatus according to any one of the preceding claims, characterised in that means (16) for controlling, in real time, the amount of air present in the replacement liquid are provided which are arranged to detect the presence of a gaseous part in the liquid before it is conveyed to the utilisation circuit and are arranged to stop the second volumetric pump (8) for conveying the replacement liquid when the gaseous part is present.

5. Apparatus according to any one of the preceding claims, characterised in that means (11) are provided for measuring the pressure of the replacement liquid, the means being arranged, in the event of an increase in the pressure, to stop the second volumetric pump (8) for conveying the liquid.

6. Apparatus according to any one of the preceding claims, characterised in that detection means (6) are provided for detecting the presence of blood in the ultrafiltrate, the means being arranged to stop the first volumetric pump (7) for the ultrafiltrate and at the same time operate an alarm device (17) when the blood is present.

7. Apparatus according to any one of the preceding claims, characterised in that means (13) are provided for computing the instantaneous loss of body weight and outputting a signal indicative thereof, the signal, suitably integrated to provide the progressive quantitative weight loss, being compared in a comparator (19) with the predetermined weight loss, the comparator being arranged to output a signal when the two magnitudes have reached the same level.

**Revendications**

1. Appareil de purification du sang utilisant le procédé d'hémofiltration ou d'hémodialysehémofiltration combinée, dans lequel l'équilibre nécessaire entre le liquide (3) prélevé du sang (ultrafiltrat) et le liquide de substitution (33) est obtenu par la régulation du débit des liquides en utilisant une première (7) et une seconde (8) pompes volumétriques pour l'ultrafiltrat et le fluide de substitution, respectivement, dont les vitesses de fonctionnement sont régulées, caractérisé en ce que des moyens (5, 40, 42, 9) sont prévus pour contrôler positivement, en temps réel, la quantité d'air présente dans le circuit de liquide, des premiers moyens sont prévus pour contrôler, en temps réel, la quantité d'air présente dans l'ultrafiltrat et sont conçus pour séparer l'air du corps liquide de l'ultrafiltrat lui-même, des seconds moyens (5) sont prévus pour détecter la réduction du corps liquide due à l'augmentation de la partie gazeuse, pour désactiver la première pompe volumétrique (7) pour l'ultrafiltrat, et pour activer une pompe aspirante (9) pour retirer les gaz excédentaires, et enfin des troisièmes moyens (4) sont prévus pour détecter le moment où le corps liquide atteint la quantité normale et pour arrêter la pompe aspirante (9) en conséquence, en remettant en route en même temps la première pompe volumétrique (7) pour l'ultrafiltrat.

2. Appareil selon la revendication 1, caractérisé en ce que la vitesse de la seconde pompe volumétrique (8) prévue pour transporter le liquide de substitution est régulée en temps réel par des moyens (12) pour calculer en continu la quantité de fluide de substitution nécessaire à tout moment par intégration de l'ultrafiltrat et pour émettre en conséquence des signaux qui effectuent la régulation du régime de la seconde pompe volumétrique (8).

3. Appareil selon l'une quelconque des revendications précédentes, caractérisé en ce que des moyens (10) pour contrôler la présence d'air dans le circuit d'ultrafiltrat sont prévus qui sont conçus pour détecter l'abaissement de la pression de l'air et sont également conçus pour arrêter la pompe d'aspiration d'air (9) et la première pompe volumétrique (7) pour l'ultrafiltrat en conséquence.

4. Appareil selon l'une quelconque des revendications précédentes, caractérisé en ce que des moyens (16) pour contrôler, en temps réel, la quantité d'air présente dans le liquide de substitution sont prévus qui sont conçus pour détecter la présence d'une partie gazeuse dans le liquide avant qu'il soit transporté vers le circuit d'utilisation et sont conçus pour arrêter la seconde pompe volumétrique (8) pour le transport du liquide de substitution quand la partie gazeuse est présente.

5. Appareil selon l'une quelconque des revendications précédentes, caractérisé en ce que des moyens (11) sont prévus pour mesurer la pression du liquide de substitution, les moyens étant conçus, en cas d'augmentation de la pression, pour arrêter la seconde pompe volumétrique (8) pour le transport du liquide.

6. Appareil selon l'une quelconque des revendications précédentes, caractérisé en ce que des moyens de détection (6) sont prévus pour détecter la présence de sang dans l'ultrafiltrat, les moyens étant conçus pour arrêter la première pompe volumétrique (7) pour l'ultrafiltrat et en même temps déclencher un dispositif d'alarme (17) quand le sang est présent.

7. Appareil selon l'une quelconque des revendications précédentes, caractérisé en ce que des moyens (13) sont prévus pour calculer la perte instantanée de poids corporel et produire un signal qui indique celle-ci, le signal, intégré de manière appropriée pour produire la perte de poids quantitative progressive, étant comparé dans un comparateur (19) à la perte de poids prédéterminée, le comparateur étant conçu pour émettre un signal lorsque les deux grandeurs ont atteint le même niveau.

**Ansprüche**

1. Vorrichtung zur Blutreinigung unter Verwendung des Verfahrens einer Hämofiltration oder einer kombinierten Hämodialyse/Hämofiltration, wobei das erforderliche Gleichgewicht zwischen der Flüssigkeit (3), die vom Blut entfernt wird (Ultrafiltrat), und der Ersatzflüssigkeit (33) durch eine Regelung der Flüssigkeitsströmungen unter Verwendung einer ersten (7) und zweiten (8) volumetrischen Pumpe für das Ultrafiltrat bzw. die Ersatzflüssigkeit erreicht wird, wobei die Betriebsgeschwindigkeiten der Pumpen geregelt werden, dadurch gekennzeichnet, daß eine Einrichtung (5, 40, 42, 9) vorgesehen ist, um im Echtzeitverfahren zwangsweise die Luftmenge zu regeln, die im Flüssigkeitskreislauf vorhanden ist, wobei eine erste Einrichtung vorgesehen ist, um im Echtzeitverfahren die Luftmenge zu regeln, die im Ultrafiltrat vorhanden ist, wobei die erste Einrichtung angeordnet ist, um die Luft vom Flüssigkeitsvolumen des Ultrafiltrats selbst zu trennen, eine zweite Einrichtung (5) vorgesehen ist, um die Abnahme des Flüssigkeitsvolumens infolge der Zunahme des gasförmigen Anteils zu ermitteln, um die erste volumetrische Pumpe (7) für das Ultrafiltrat außer Betrieb zu setzen und eine Saugpumpe (9) in Betrieb zu setzen, um die überschüssigen Gase zu beseitigen, sowie schließlich eine dritte Einrichtung (4) vorgesehen ist, um zu ermitteln, wenn das Flüssigkeitsvolumen die normale Menge erreicht, und die Saugpumpe (9) infolgedessen anzuhalten, wobei gleichzeitig die erste volumetrische Pumpe (7) für das Ultrafiltrat wieder in Betrieb gesetzt wird.

2. Vorrichtung gemäß Anspruch 1, dadurch gekennzeichnet, daß die Geschwindigkeit der zweiten volumetrische Pumpe (8), die angeordnet ist, um die Ersatzflüssigkeit zu fördern, im Echtzeitverfahren mit einer Einrichtung (12) geregelt wird, um fortlaufend die Menge der Ersatzflüssigkeit, die in jedem Moment notwendig ist, durch eine Integration des Ultrafiltrats zu berechnen und infolgedessen Signale abzugeben, die eine Regelung der Geschwindigkeit der zweiten Pumpe (8) bewirken.

3. Vorrichtung gemäß jedem der bisherigen Ansprüche, dadurch gekennzeichnet, daß eine Einrichtung (10) vorgesehen ist, um das Vorhandensein von Luft im Ultrafiltratkreislauf zu regeln, wobei die Vorrichtung so angeordnet ist, um das Absinken des Luftdrucks abzutasten, und weiters so angeordnet ist, um infolgedessen die Luftsaugpumpe (9) und die erste volumetrische Pumpe (7) für das Ultrafiltrat anzuhalten.

4. Vorrichtung gemäß jedem der bisherigen Ansprüche, dadurch gekennzeichnet, daß eine Einrichtung (16) vorgesehen ist, um im Echtzeitverfahren die Luftmenge zu regeln, die in der Ersatzflüssigkeit vorhanden ist, wobei die Einrichtung so angeordnet ist, um das Vorhandensein eines gasförmigen Anteils in der Flüssigkeit zu ermitteln, bevor diese zum Nutzkreislauf geführt wird, und weiters so angeordnet ist, um die zweite volumetrische Pumpe (8) für die Förderung der Ersatzflüssigkeit anzuhalten, wenn der gasförmige Anteil vorhanden ist.

5. Vorrichtung gemäß jedem der bisherigen Ansprüche, dadurch gekennzeichnet, daß eine Einrichtung (11) vorgesehen ist, um den Druck der Ersatzflüssigkeit zu messen, wobei diese Einrichtung angeordnet ist, um bei einem Druckanstieg die zweite volumetrische Pumpe (8) für die Förderung der Flüssigkeit anzuhalten.

6. Vorrichtung gemäß jedem der bisherigen Ansprüche, dadurch gekennzeichnet, daß eine Fühlereinrichtung (6) vorgesehen ist, um das Vorhandensein von Blut im Ultrafiltrat festzustellen, wobei die Einrichtung so angeordnet ist, um die erste volumetrische Pumpe (7) für das Ultrafiltrat anzuhalten und gleichzeitig eine Alarmeinrichtung (17) in Betrieb zu setzen, wenn Blut vorhanden ist.

7. Vorrichtung gemäß jedem der bisherige Ansprüche, dadurch gekennzeichnet, daß eine Einrichtung (13) vorgesehen ist, um den momentanen Verlust an Körpergewicht zu berechnen und ein Signal abzugeben, das dies anzeigt, wobei das Signal, das geeignet integriert wird, um den fortschreitenden mengenmäßigen Gewichtsverlust zu liefern, in einem Vergleicher (19) mit dem vorgegebenen Gewichtsverlust verglichen wird, wobei der Vergleicher so angeordnet ist, um ein Signal abzugeben, wenn die beiden Größen den gleichen Pegel erreicht haben.